# EUROPEAN PATENT SPECIFICATION

(11) **EP 1 636 590 B1**
(45) Date of publication and mention of the grant of the patent: **14.01.2009**
(21) Application number: 04740217.7
(22) Date of filing: 23.06.2004
(51) Int. Cl.: G01N 33/68

(54) **MODULATION OF OSTEOBLAST ACTIVITY BY FHL2**
MODULIERUNG DER OSTEOBLAST-AKTIVITÄT DURCH FHL2
MODULATION DE L'ACTIVITE DES OSTEOBLASTES PAR FHL2

(30) Priority: 24.06.2003 EP 03013451
(43) Date of publication of application: 22.03.2006
(73) Proprietor: Universitätsklinikum Freiburg, 79106 Freiburg (DE)
(72) Inventor: SCHÜLE, Roland, 79367 Weisweil (DE); GÜNTHER, Thomas, 79108 Freiburg (DE); MÜLLER, Judith, 79108 Freiburg (DE)
(74) Representative: Kalhammer, Georg
(86) International application number: PCT/EP2004/006798
(87) International publication number: WO 2005/001482

(56) References cited:
- WO-A-99/51727
- US-B1- 6 333 312
- MÜLLER JUDITH M ET AL: "The transcriptional coactivator FHL2 transmits Rho signals from the cell membrane into the nucleus." THE EMBO JOURNAL. ENGLAND 15 FEB 2002, vol. 21, no. 4, 15 February 2002 (2002-02-15), pages 736-748, XP001156130 ISSN: 0261-4189 cited in the application
- AMAAR YOUSEF G ET AL: "Insulin-like growth factor-binding protein 5 (IGFBP-5) interacts with a four and a half LIM protein 2 (FHL2)" JOURNAL OF BIOLOGICAL CHEMISTRY, vol. 277, no. 14, 5 April 2002 (2002-04-05), pages 12053-12060, XP001153236 ISSN: 0021-9258
- JOHANNESSEN MONA ET AL: "Activation of the coactivator four-and-a-half-LIM-only protein FHL2 and the c-fos promoter through inhibition of protein phosphatase 2A." BIOCHEMICAL PHARMACOLOGY, vol. 65, no. 8, 15 April 2003 (2003-04-15), pages 1317-1328, XP001153237 ISSN: 0006-2952
- CHAN K K ET AL: "Molecular cloning and characterization of FHL2, a novel LIM domain protein preferentially expressed in human heart" GENE: AN INTERNATIONAL JOURNAL ON GENES AND GENOMES, ELSEVIER SCIENCE PUBLISHERS, BARKING, GB, vol. 210, no. 2, 14 April 1998 (1998-04-14), pages 345-350, XP004118187 ISSN: 0378-1119
- KONG Y ET AL: "Cardiac-specific LIM protein FHL2 modifies the hypertrophic response to beta-adrenergic stimulation." CIRCULATION. UNITED STATES 5 JUN 2001, vol. 103, no. 22, 5 June 2001 (2001-06-05), pages 2731-2738, XP001153299 ISSN: 1524-4539 cited in the application
- LAI C F ET AL: "Four and a half Lim protein 2 (FHL2) stimulates osteoblast proliferation and differentiation." JOURNAL OF BONE AND MINERAL RESEARCH, vol. 17, no. Suppl 1, September 2002 (2002-09), page S129, XP009020682 Twenty-Fourth Annual Meeting of the American Society for Bone and Mineral Research;San Antonio, Texas, USA; September 20-24, 2002 ISSN: 0884-0431 (ISSN print)
- VAUGHAN TANYA ET AL: "Alleles of RUNX2/CBFA1 gene are associated with differences in bone mineral density and risk of fracture." JOURNAL OF BONE AND MINERAL RESEARCH, vol. 17, no. 8, August 2002 (2002-08), pages 1527-1534, XP009021473 ISSN: 0884-0431 (ISSN print)

## Description

The present invention provides means and methods for regulating the activity of osteoblasts. The invention further relates to methods for identifying compounds that are useful in the treatment of osteoporosis.

Bone is an organ of special importance offering resistance to mechanical stress and protecting internal organs from trauma. Bone is built by only three different cell types. During embryonic development chondrocytes produce a blueprint of cartilage for most bones. Successively, the cartilage is replaced by bone substance. This is an extracellular matrix mostly produced by osteoblasts which calcifies under its control. Therefore osteoblasts represent the bone forming cells. The formation of bone is yet not static. During the entire life osteoclasts degrade bone substance which subsequently will be replaced by the activity of osteoblasts thereby absorbing the varying burden of the skeleton (Karsenty and Wagner, 2002). An equilibrium of the activity of osteoblasts and osteoclasts exists in a full-grown human before the entrance into old age. However, if the number and/or activity of osteoblasts decreases or if the number and/or activity of osteoclasts increases expanded bone loss will be the outcome (osteoporosis). The reduced bone substance causes an increased risk for fractures (Teitelbaum, 2000).

Osteoporosis can have quite different reasons. The most important one is the increase of the osteoclast cell population and its activity in postmenopausal women due to decreased estrogen synthesis. Besides that, differentiation, activity and apoptosis of osteoclasts as well as the presence of some extracellular matrix proteins play an important role. The knowledge about loss of bone substance caused by decreased osteoblast activity is still surprisingly small. This is very well underlined by the lack of supporting animal models.

Osteoporosis is of outstanding individual and economic significance. It is the most prominent degenerative disease in the western world. The economic damage caused by fractures due to osteoporosis only in the USA is assessed to be about 10 to 15 billion $ (NIH Consensus Development Panel on Osteoporosis Prevention, 2001).

Approaches to fight osteoporosis on the pharmacological level lie in the increase of serum calcium by supplementation of nutrition. Also specific manipulation of the estrogen signaling cascade by administration of estrogen or the modulation of the estrogen receptor (e.g. Raloxifene) is widely used in females. Inhibition of bone turnover by bisphosphonates (e.g. Alendronate) or the inhibition of bone resorption by calcitonin can be an alternative strategy.

Most of the known therapies try to inhibit osteoclast activity, e.g., stop bone resorption and continuing damage. Hence, there is an ongoing need for a therapy that could lead to the reconstruction of bone substance resembling normal bone mineral density.

The inventors surprisingly found that Fhl2 significantly increases the transcriptional activity of the gene Runx2. In addition, the interaction of Fhl2 protein and Runx2 protein regulates the activity of target genes, e.g., osteocalcin, and therefore the activity of osteoblasts.

As used herein, the term "activity of osteoblasts" denotes the capability of osteoblasts to form extracellular matrix. This activity can be determined *in vitro* by the following assay: Synthesis by the cells of extracellular matrix is induced by adding 5 mM β-glycerophosphate and 100 µg/ml medium ascorbic acid. After incubation, the amount of calcified matrix is determined by von Kossa staining and quantification of the stained area (Bancroft, J.D. and A. Stevens. 1996. Theory and practice of histological techniques. Churchil Livingston, New York, NY).

Osteoblast activity can be determined in vivo as described in Example 1.

"Four and a Half LIM Domains 2" (Fhl2) belongs to a subclass of the "LIM-only" proteins of the super-family of LIM-domain proteins. Family members are characterized by the presence of a single or several LIM domains. LIM proteins play an important role in determination, differentiation and proliferation of cells. Fhl2 as well as Fhl1, -3, -4 and "Activator of Crem in Testis" (Act) are characterized by four complete and a single half LIM domain. The LIM domain is a cysteine-rich motif that can bind two zinc ions and mediates protein-protein interactions.

It has been described that insulin-like growth factor binding protein 5 (IGFBP-5) interacts with FHL2. Northern blot analysis showed that Fhl2 was expressed in human osteoblasts (The Journal of Biological Chemistry, Vol. 277, No. 14, issue of April 5, pp. 12053 - 12060, 2002).

Johannessen et al. (Biochemical Pharmacology, 65, pp. 1317 - 1328, 2003) report that Fhl2 can be activated by inhibition of protein phosphatase 2A (PP2A).

Chan et al. (Gene: An International Journal of Genes and Genomes, 210, pp. 345 - 350, 1998) report that Fhl2 is preferentially expressed in the human heart.

WO 99/51727 A2 discloses nucleic acid sequences of normal ovary tissue.

Lai et al. (Journal of Bone and Mineral Research, ASBMR, 24th Annual Meeting, S129, Abstract 1017, September 2002) describe that Fhl2 stimulates osteoblast proliferation and differentiation.

US patent No. 6,333,312 describes treatments of bone diseases such as osteoporosis and compounds/morphogens useful therefor.

Vaughan et al. (Journal of Bone and Mineral Research, Vol. 17, No. 8, pp. 1527 - 1534, 2002) describe studies on the role of RUNX2 as a regulator of osteoblast function. Alleles of RUNX2 were found to be associated with differences in bone mineral density.

Besides expression in the heart muscle FHL2 could be detected in the epithelial cells of the prostate where it very well co-localizes with the activated androgen receptor (AR) in the nucleus (Müller et al. 2002). The AR belongs to a family of steroid receptors which are capable of directly binding to so called response elements in the promoter of target genes.

Their transcriptional activity can be influenced by cofactors. Fhl2 can directly bind to AR thereby functioning as a tissue specific coactivator (Müller et al. 2000). The translocation of Fhl2 from the cytoplasm into the nucleus and the super-activation of the transcriptional activity of the AR is specifically triggered by Rho-GTPases. This way, extracellular signals can be translated into altered gene expression via the Rho-signaling cascade and Fhl2. The aberrant regulation of the AR activity plays a decisive role in the formation and progression of tumors in the prostate. Indeed, over-expression of Rho-GTPases in prostate carcinomas and the correlation of nuclear Fhl2 localization with the malignancy of the tumor has been shown. (Müller et al., 2002).

The present invention therefore relates to a method for identifying a compound that promotes the activity of osteoblasts, comprising:
(a) contacting a test compound with at least one cell *in vitro*;
(b) determining the level of interaction between Fhl2 protein and Runx2 protein in the cell(s);
(c) comparing the level of interaction determined in (b) to the level of interaction between Fhl2 protein and Runx2 protein in at least one control cell that has not been contacted with the test compound; and
(d) selecting the compound if the level of interaction measured in (b) is significantly different from that in the control cell(s).

The method may further comprise one or more of the following steps:
- determining whether the test compound is capable of promoting the activity of osteoblasts; and optionally selecting the test compound if it is capable of promoting the activity of osteoblasts;
- determining whether the test compound is capable of modulating the bone formation rate in a non-human test animal; and optionally selecting the test compound if it is capable of modulating the bone formation rate in a non-human test animal;
- determining whether the test compound is capable of promoting the formation of extracellular bone matrix in a non-human test animal; and optionally selecting the test compound if it is capable of promoting the formation of extracellular bone matrix in a non-human test animal;
- determining whether the test compound is capable of increasing the expression of the Runx2 gene; and optionally selecting the test compound if it is capable of increasing the expression of the Runx2 gene;
- using the test compound for the manufacture of a medicament for the treatment of bone disease, e.g., osteoporosis.

The term "activity of the Fhl2 gene or Fhl2 protein" includes but is not limited to, the expression of the Fhl2 gene (e.g., transcription and/or translation); the ability of the Fhl2 protein to translocate into the cell nucleus; the capability of the Fhl2 protein to interact with the Runx2 protein; the capability of the Fhl2 protein to stimulate the expression of other genes; and the like.

In one embodiment the test compound is selected if the activity of the Fhl2 gene or Fhl2 protein is significantly higher than that of the control. In another embodiment, the test compound is selected if the activity of the Fhl2 gene or Fhl2 protein is significantly lower than that of the control.

Suitable cell types that can be used include, but are not limited to, primary osteoblasts; MC3T3-E1 cells, ROS17 cells; and U2-OS cells. Preferably, the cells are osteoblasts, e.g., primary osteoblasts. Osteoblasts can be obtained as primary cells from the calvaria of newborn mice and subsequently cultured. By adding 5 mM glycerophosphate and 100 µg/ml medium ascorbic acid the production of calcifying extracellular matrix can be induced (Zhang et al. 1997; Journal of Biological Chemistry 272, 110-116). The cell line MC3T3-E1 is an established mouse osteoblast cell line.

In a specific embodiment, cells, preferably animal cells, and more preferably mammalian cells that have been manipulated to be missing all or essentially all of an activity of the Fhl2 protein can be used. Such cells may be derived from a transgenic non-human animal described infra. The transgenic non-human animal is characterized by a decreased level of expression of the Fhl2 gene relative to that of the corresponding wild-type animal. The transgenic non-human animal may be an Fhl2-deficient knockout mouse.

Usually, more than one cell is used. In that case, a population of cells in cell culture may be grown under suitable conditions. The test compound can be added to the population of cells, e.g., by adding it to the cell culture medium. Methods of culturing cells are known to those skilled in the art. The control cells are usually of the same cell type as the cells to which the test compound has been added. They are usually cultured under substantially identical conditions except for addition of the test compound.

Further described herein is a method which comprises:
(a) contacting a test compound with at least one cell *in vitro;*
(b) measuring the level of *Fhl2* expression in the cell(s);
(c) comparing the level of *Fhl2* expression measured in (b) to the level of *Fhl2* expression in at least one control cell that has not been contacted with the test compound; and
(d) selecting the compound if the level of *Fhl2* expression measured in (b) is higher than that in the at least one control cell.

The step of determining the level of expression of the Fhl2 gene includes, but is not limited to, measuring the amount and/or concentration of Fhl2 mRNA and/or measuring the amount and/or concentration of Fhl2 protein in cells or tissue.

The amount of Fhl2 mRNA in cells or tissue can be measured by methods known to those skilled in the art, e.g., by RT-PCR (Du et al., 2002, Biochimica et Biophysica Acta 1577, 93-101) or a Northern or Western Blot protocol (Müller et al. 2000; EMBO J. 19:359-369). The nucleotide sequence of human Fhl2 as shown in SEQ ID NO:1 can be used to design suitable primers and/or probes capable of hybridizing to Fhl2 mRNA or Fhl2 cDNA under stringent conditions as described herein. It is contemplated that nucleic acid segments that comprise a sequence region that consists of at least a 14 nucleotide long contiguous sequence that has the same sequence as, or is complementary to, a 14 nucleotide long contiguous sequence of SEQ ID NO:1 will find particular utility. Longer contiguous identical or complementary sequences, e.g., those of at least 20, 30, 40, 50, 100, 200, 500 and even up to full length sequences will also be of use in certain embodiments. This embodiment is particularly useful if human cells or human tissue is used. If cells or tissue from non-human origin are employed the corresponding non-human nucleotide sequence of the Fhl2 gene can be used for designing suitable primers or probes. For example, when mouse cells are used, it is preferred that the primers or probes are derived from the mouse Fhl2 sequence (Accession No. NM_010212).

The stringent conditions referred to herein include, for example, hybridization at 20 mM to 40 mM NaCl at a temperature of about 50°C to about 70°C, preferably at about 60°C to 65°C.

The amount of Fhl2 protein in cells or tissue can be measured by methods generally known in the art, e.g., by immunochemical methods using antibodies recognizing the Fhl2 protein. Such methods include, but are not limited to, Western blots, dot blots, ELISA protocols, histochemical methods, and the like. Antibodies recognizing Fhl2 protein can be prepared and optionally purified using methods known to those skilled in the art. Methods for preparing and isolating polyclonal and monoclonal antibodies are well known in the art. See, for example, Current Protocols in Immunology, Cooligan et al. (eds.), National Institutes of Health, John Wiley and Sons, Inc., 1995; Sambrook et al., Molecular Cloning: A Laboratory Manual, Second Edition, Cold Spring Harbor, NY, USA, 1989; and Hurrell, J.G.R., Ed., Monoclonal Hybridoma Antibodies: Techniques and Applications, CRC Press, Inc., Boca Raton, Florida, USA, 1982. The amino acid sequence of human Fhl2 protein as shown in SEQ ID NO:2 can be used in order to design suitable antigens for the generation of antibodies. If cells from non-human origin are employed suitable antigens can be derived from the amino acid sequence of the corresponding non-human Fhl2 protein. Monoclonal and polyclonal antibodies against Fhl2 protein have been described in Müller et al. (2000) EMBO J. 19, 359-369 and in Müller et al. (2002) EMBO J. 21, 736-748.

Independently of step (a) and (b), the level of Fhl2 expression in control cells can be determined. The control cells have not been contacted with the test compound. The control cells have preferably been cultured under conditions which are identical to the conditions under which the cells that have been contacted with the test compound have been cultured, except for addition of the test compound. Usually, the compound is selected if the level of Fhl2 expression measured in (b) is significantly higher than that in the control cells.

Preferably, the compound is selected if the level of Fhl2 expression measured in (b) is at least 10% or at least 20% or at least 50% or at least 100% higher than that in the control cells.

Further described herein is a method for identifying a compound that promotes the activity of osteoblast, comprising:
(a) contacting at least one cell with a test compound *in vitro;*
(b) measuring the amount of Fhl2 protein in the nucleus of the cell(s);
(c) comparing the amount of Fhl2 protein measured in (b) to the amount of Fhl2 protein in the nucleus of at least one control cell that has not been contacted with the test compound; and
(d) selecting the compound if the level of Fhl2 protein measured in (b) is higher than that (in the control cell(s).

The amount of Fhl2 protein in the nucleus of cells may be determined by one of the following methods: The intracellular localization of Fhl2 can be detected by immune histochemistry using an anti-Fhl2 antibody as described in Müller et al., 2002, EMBO J. 21:736-748. In another embodiment, the amount of Fhl2 in a nuclear extract and in a total cell extract can be determined (see, e.g., Müller et al., 2002, EMBO J. 21:736-748).

The present invention relates to a method for identifying a compound that promotes the activity of osteoblasts comprises:
(a) contacting at least one cell with a test compound *in vitro;*
(b) determining the level of interaction between Fhl2 protein and Runx2 protein in the at least one cell;
(c) comparing the level of interaction determined in (b) to the level of interaction between Fhl2 protein and Runx2 protein in at least one control cell that has not been contacted with the test compound; and
(d) selecting the compound if the level of interaction measured in (b) is significantly different from that in the at least one control cell.

Step (b) includes methods known to those skilled in the art, e.g., co-immunoprecipitation and pulldown assays as described herein (see Example 2). Direct or indirect interaction of Fhl2 and Runx2 can be determined in a transfection assay wherein a reporter gene is employed. For example, a reporter gene operably linked to a promoter which binds to Runx2 may be transfected together with Runx2 DNA and Fhl2 DNA (See Examples).

In a specific embodiment, the compound is selected if the level of interaction determined in (b) is significantly higher than that in the at least one control cell. Preferably, the level of interaction determined in (b) should be at least 10% or at least 20% or at least 50% or at least 100% higher than that in the at least one control cell.

In another specific embodiment, the compound is selected if the level of interaction measured in (b) is significantly lower than that in the at least one control cell. Preferably, the level of interaction determined in (b) should be less than 90% or 80% or 50% or 25% of that in the at least one control cell.

Further described herein is a compound that is useful in the treatment of a bone disease wherein the compound is capable of promoting osteoblast activity by enhancing the expression of the Fhl2 gene, promoting the translocation of Fhl2 protein in the nucleus and/or modulating the interaction between Fhl2 protein and Runx2 protein. The compound is obtainable by a method described herein. The compound may be capable of enhancing signals mediated by Rho proteins. Examples of compounds that influence the Rho signalling pathway include, but are not limited to, lysophosphatidic acid (LPA) and sphingosine-1-phosphate (SPP).

The invention further concerns the use of a compound according to the invention for the manufacture of a medicament for the treatment of a bone disease, e.g., osteoporosis.

Another aspect of the present invention is a method of diagnosing a bone disease, comprising:
(a) determining *in vitro* the level of expression of the *Fhl2* gene in tissue from an individual; and
(b) comparing the level determined in (a) to the level of expression of the *Fhl2* gene in control tissue;
   so that if the level determined in (a) is lower than that of the control, the individual is diagnosed as exhibiting the bone disease.

The level of Fhl2 expression may be determined as described supra, e.g., by measuring the amount/concentration of mRNA in the cells or by measuring the amount/concentration of Fhl2 protein present in the cells.

The determination can be carried out using a tissue sample derived from an individual. Preferably, the tissue sample comprises bone material, more preferably the tissue sample comprises osteoblasts. The tissue sample may be obtained through biopsy from an individual. In a specific embodiment, osteoblasts are enriched after having obtained the biopsy and prior to determining the level of expression of the Fhl2 gene.

Independently of step (a), one can determine *in vitro* the level of expression of the Fhl2 gene in tissue or cells from a healthy control individual. Preferably, the type of tissue or cells to be examined as control is the same type of tissue or cells as that used in step (a). It is important that the control individual does not suffer from a bone disease, e.g., osteoporosis.

From the amount of Fhl2 mRNA or protein one can derive the concentration of Fhl2 mRNA or protein, given as g/(number of cells), mol/(number of cells), g/(g cell mass), mol/(g cell mass), or the like.

Usually, the individual is diagnosed as exhibiting the bone disease, if the level determined in step (a) is lower than that of the control. In specific embodiments, the individual is diagnosed as exhibiting the bone disease, if the level of Fhl2 expression (e.g., the amount or concentration of the Fhl2 mRNA or the amount or concentration of the Fhl2 protein) determined in (a) is less than 90% or 80% or 70% or 60% or 50% of the level of Fhl2 expression of the control.

Preferably, the bone disease to be diagnosed is characterized by a decreased bone mass relative to that of non-diseased bone. Most preferably, the bone disease to be diagnosed is osteoporosis.

The present invention further relates to the use of an Fhl2 nucleic acid for the manufacture of a medicament for the treatment of bone disease. As used herein, the phrase "Fhl2 nucleic acid" denotes one of the following nucleic acids:
(i) Polynucleotides comprising a sequence which has an identity of at least 50% to the sequence as shown in SEQ ID NO:1. Preferably, the identity is at least 60%, more preferably at least 70%, still more preferably at least 80%, still more preferably at least 90%. Most preferably, the polynucleotide comprises a sequence as shown in SEQ ID NO:1.
(ii) Polynucleotides hybridizing to the sequence as shown in SEQ ID NO:1 under stringent conditions.
(iii) Polynucleotides comprising a sequence which encodes a polypeptide having an amino acid sequence which has an identity of at least 50% to the amino acid sequence as shown in SEQ ID NO:2. Preferably, the identity is at least 60%, more preferably at least 70%, still more preferably at least 80%, still more preferably at least 90%. Most preferably, the polynucleotide comprises a sequence which encodes a polypeptide having an amino acid sequence as shown in SEQ ID NO:2.

In one embodiment the bone disease is characterized by a decreased bone mass relative to that of non-diseased bone. The bone disease may be selected from the group consisting of osteoporosis, osteopenia, and Paget's disease. Preferably, the bone disease is osteoporosis.

The Fhl2 nucleic acid may be used in a gene therapy protocol to increase the activity of osteoblasts. If an individual has a mutated or absent Fhl2 gene or shows a decreased level of Fhl2 expression, the Fhl2 nucleic acid can be introduced into the cells of the individual. In one embodiment, a gene encoding a Fhl2 polypeptide is introduced in vivo in a viral vector. Such vectors include an attenuated or defective DNA virus, such as, but not limited to, herpes simplex virus, papilloma virus, Epstein-Barr virus, adenovirus, adeno-associated virus, and the like. The effective viruses which entirely or almost entirely lack viral genes are preferred. A defective virus is not effective after the introduction into a cell. Use of defective viral vectors allows for administration to cells in a specific, localized area, without concern that the vector can infect other cells. Examples of particular vectors include, but are not limited to, a defective herpes simplex virus 1 vector (Kaplitt et al., Molec. Cell. Neurosci. 2:320-30, 1991); an attenuated adenovirus vector, such as the vector described by Stratford-Perricaudet et al., J. Clin. Invest. 90:626-30, 1992; and a defective adeno-associated virus vector (Samulski et al., J. Virol. 61:3096-101, 1987; Samulski et al., J. Virol. 63:3822-8, 1989).

In another embodiment, a Fhl2 nucleic acid can be introduced in a retroviral vector, e.g., as described in Anderson et al., U.S. patent No. 5,399,346; Mann et al. Cell 33:153, 1983; Temin et al., U.S. patent No. 4,650,764; Temin et al., U.S. patent No. 4,980,289; Markowitz et al., J. Virol. 62:1120, 1988; Temin et al., U.S. patent No. 5,124,263; WO 95/07358; and Kuo et al., Blood 82:845, 1993. Alternatively, the vector can be introduced by lipofection in vivo using liposomes. Synthetic cationic lipids can be used to prepare liposomes for in vivo transfection of a gene encoding a marker (Felgner et al., Proc. Natl. Acad. Sci. USA 84:7413-7, 1987; Mackey et al., Proc. Natl. Acad. Sci. USA 85:8027-31, 1988). The use of lipofection to introduce exogenous genes into specific organs in vivo has certain practical advantages. Molecular targeting of liposomes to specific cells represents one area of benefit. More particularly, directing transfection to particular cells represents one area of benefit. For instance, directing transfection to osteoblasts would be particularly advantageous. Lipids may be chemically coupled to other molecules for the purpose of targeting. Targeted peptides (e.g., hormones or neurotransmitters), proteins such as antibodies, or non-peptide molecules can be coupled to liposomes chemically.

It is possible to remove the target cells from the body; to introduce the vector as a naked DNA plasmid; and then to re-implant the transformed cells into the body. Naked DNA vectors for gene therapy can be introduced into the desired host cells by methods known in the art, e.g., transfection, electroporation, microinjection, transduction, cell fusion, DEAE dextran, calcium phosphate precipitation, use of a gene gun or use of a DNA vector transporter. See, e.g. Wu et al., J. Biol. Chem. 267:963-7, 1992; Wu et al., J. Biol. Chem. 263:14621-4, 1988.

In another embodiment, the invention pertains to a method for identifying Fhl2-regulated target genes. The method may comprise the step of comparing a cDNA pool from a first population of cells with a cDNA pool from a second population of cells wherein in the first population of cells Fhl2 transcription or nuclear translocation has been induced. In the second population of cells Fhl2 transcription or nuclear translocation has not been induced. Microarrays may be used for comparing the cDNA pools.

A genetically modified non-human animal characterized by a decreased level of expression of the Fhl2 gene relative to that of the non-modified non-human animal may be used as an osteoporosis animal model. The level of expression of the Fhl2 gene in the modified non-human animal may be less than 90%, preferably less than 75%, more preferably less than 50% and still more preferably less than 25% of the level of Fhl2 expression in the non-modified non-human animal. The amount or concentration of Fhl2 mRNA or Fhl2 protein in bone tissue may be a measure for the level of expression of the Fhl2 gene.

Most preferably, the non-human animal completely lacks expression of functional Fhl2 protein. In that embodiment, the Fhl2 gene may have been deleted or rendered non-functional.

It is also preferred that the non-human animal is a non-human mammal, more preferably the non-human animal is a rodent, most preferably, the non-human animal is a mouse.

The non-human animal may have been manipulated to be missing all or essentially all of an activity of one or more specific gene/allele products. In a preferred embodiment, the non-human animal has been manipulated so as not to express functional Fhl2 protein.

In addition, according to the invention cells, preferably animal cells, and more preferably mammalian cells that have been manipulated to be missing all or essentially all of an activity of the Fhl2 protein, may be useful as osteoporosis model systems. The cells may be human or non-human cells. Preferably, the cells are bone cells, e.g., osteoblasts.

The invention provides methods of making and using these cells and non-human animals. The knockout animals and/or corresponding cells of the present invention can be manipulated to be incapable of expressing a functional protein from one or more specified alleles and/or genes by any means known in the art. For example, a knockout animal and/or corresponding cell can be manipulated to comprise a disruption in an endogenous allele and/or gene, e.g., the Fhl2 gene, thereby preventing the expression of the corresponding functional protein. Alternatively, a knockout animal and/or corresponding cell can be manipulated to comprise a dominant mutant allele. Fhl2 knockout mice have been described in Kong et al. 2001, Circulation 103, 2731-2738.

In yet another embodiment, a knockout animal and/or corresponding cell can be treated with one or more antisense nucleic acids for one or more specific gene(s) thereby inhibiting the expression of the specific gene(s). In still another embodiment, the gene(s) encoding the specific functional protein(s) can be constructed such that the expression of the protein(s) is under the control of an inducible promoter, and this expression is conditionally repressed. In still another embodiment, the cell and/or nun-human animal is treated with/administered inhibitory compound(s) that prevent the expression and/or activity of the Fhl2 protein.

The invention includes the use of a non-human knockout animal that comprises a homozygous disruption in its endogenous Fhl2 gene as an osteoporosis model.

The non-human animal preferably shows decreased bone formation rate relative to that of the corresponding wild-type animal. The bone formation rate may be decreased by at least 5%, preferably at least 10%, most preferably at least 15%, compared to the corresponding wild-type animal. The bone formation rate may be determined as described in Example 1.

The non-human animal preferably shows a decrease of the amount of the calcified extracellular matrix. This decrease may concern the vertebral bodies of the non-human animal. The amount of the calcified extracellular matrix may be decreased by at least 10%, preferably by at least 20%, more preferably by at least 25%, most preferably by at least about 30%, compared to the amount of the calcified extracellular matrix in a corresponding wild-type animal. The decrease can be present in male or in female animals.

According to the invention also a cell from the transgenic non-human animal of the invention may be useful. Preferably the cell is from the knockout mouse and has the same genetic background as the knockout mouse.

"Gene targeting" is a type of homologous recombination that occurs when a fragment of genomic DNA is introduced into a mammalian cell and that fragment locates and recombines with endogenous homologous sequences as exemplified in US patent No. 5,464,764 and US patent No. 5,777,195.

As used herein, the term "knockout animal" denotes an animal that has been manipulated to be irreversibly missing all or "essentially all" of an activity of one or more specific gene/allele product(s) relative to the corresponding wild type animal. In a particular embodiment of this type, the knockout animal contains within its genome a specific gene/allele that has been inactivated by a method such as gene targeting. As used herein, the term "knockout animal" can therefore include the heterozygote animal (e.g., one defective allele and one wild-type allele), a homozygous animal (e.g., two defective alleles) or an animal having more than one gene inactivated. In a particular embodiment of the present invention, a knockout animal is a knockout mouse that has both alleles of the Fhl2 gene inactivated.

The non-human animal described supra can be used as an in vivo osteoporosis model. The non-human animals offer the unique possibility to test the effect of pharmacological substances on the activity of osteoblasts and therefore their efficacy in osteoporosis treatment. The invention relates to the use of an Fhl2-deficient non-human animal for the development of a medicament for the treatment of bone disease, e.g., osteoporosis.

The present invention offers a completely new approach to specifically fight osteoporosis by the possibility of the well-defined modulation of osteoblast activity. Contrary to the known therapies (inhibition of osteoclast activity; that is stop of bone resorption and continuing damage) the present invention offers the possibility to develop methods and substances that could lead to the reconstruction of bone substance resembling normal bone mineral density. So far only one transcription factor (Runx2) is known to modulate activity of fully differentiated osteoblasts (Ducy et al., 1999). The inventors showed that Fhl2 significantly increases the transcriptional activity of Runx2. The interaction of Fhl2 and Runx2 regulates the activity of target genes (e.g. osteocalcin) and therefore the activity of osteoblasts. This observation is corroborated *in vivo* by the osteoporosis phenotype of *Fhl2*-deficient mice. Fhl2^{-/-} mice exhibit a decreased osteoblast activity.

The various embodiments of the invention described herein may be combined.

The pharmacological intervention in the Fhl2-mediated signaling cascade offers a new attempt to regulate osteoblast activity directly. Possible pharmacological targets are:
1.) Protein-protein interaction required for the activation of Fhl2 (e.g. modulation of the Runx2-Fhl2 interaction).
2.) Substances modulating the translocation of Fhl2 from the cytoplasm into the nucleus (e.g. modulators of the Rho signaling cascade; Müller et al., 2002).
3.) Fhl2-regulated target genes in osteoblasts.
4.)The *Fhl2*-deficient mice are a new in vivo osteoporosis model. Fhl2^{-/-} mice offer the unique possibility to test the effect of pharmacological substances in regards to the activity of osteoblasts and therefore their efficacy in osteoporosis treatment.

Figure 1: Expression of FHL2 in bone of adult mice.
   The endogenous expression of FHL2 in bone, bone marrow and periosteum of the tibia is reflected through the LacZ stain in a heterozygote mouse. The LacZ gene is under the control of the endogenous Fhl2 promoter.
Figure 2: Decreased calcification of the extracellular matrix in Fhl2^{-/-} bone.
   Von Kossa stain of vertebral bodies from an adult wild-type (left) and Fhl2^{-/-} (right) mouse. The comparison reflects a decrease of 30% in the calcified extracellular matrix (black) in mutant mice.
Figure 3: Number of osteoblasts and osteoclasts.
   Despite osteoporosis, the number of osteoblasts and osteoclasts is not altered in adult vertebral bodies of wild-type and Fhl2^{-/-} mice.
Figure 4: Activity of osteoclasts is not increased.
   Quantification of osteoclast activity by measurement of the corresponding products of bone decomposition in the urine. The amount of pyridinium crosslinks is determined in relation to the total protein content. Osteoclast activity is not altered in *Fhl2*-deficient mice compared to wild-type animals.

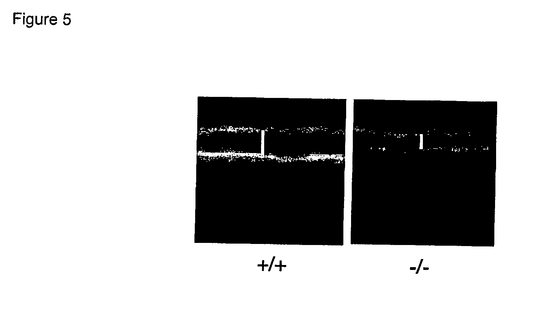

Figure 5: Decreased bone formation rate in Fhl2^{-/-} mice.
   Dynamic histomorphometric analysis after in vivo labeling with the fluorescent dye calcein in an interval of 7 days. The distance (yellow bar) of the mineralization fronts (green) in representative sections of the tibia of adult wild-type and Fhl2^{-/-} mice reflect osteoblast activity. The osteoblast activity is decreased by 19% in mutant mice.

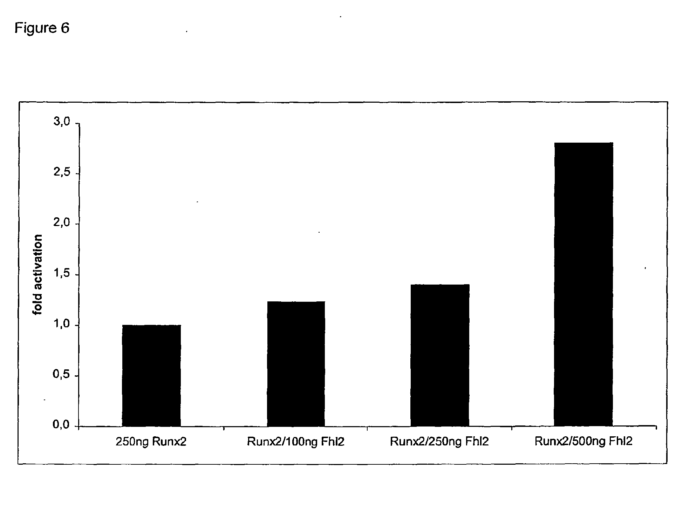

Figure 6: Fhl2 is a coactivator for Runx2 using an artificial promoter.
   Concentration-dependent increase of the transcriptional activity of Runx2 by Fhl2 in transient transfections in Cos-7 cells.

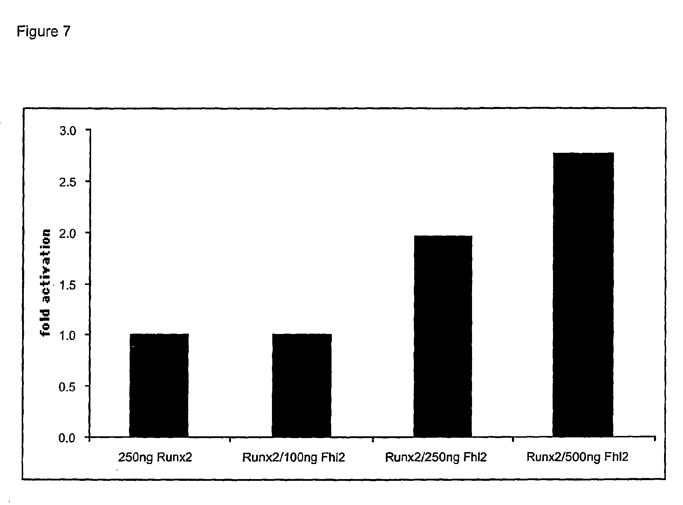

Figure 7: Fhl2 enhances the effect of Runx2 on the human osteocalcin promoter.
   Concentration-dependent increase of the transcriptional activity of Runx2 by Fhl2 in transient transfections in Cos-7 cells. The human Osteocalcin promoter contains two binding sites for Runx2.

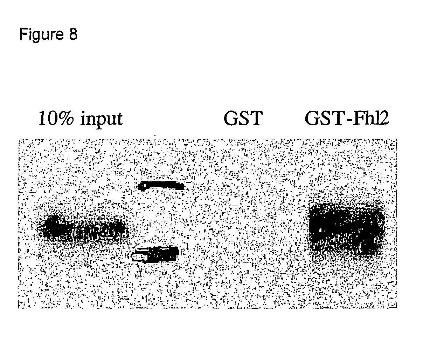

Figure 8: Physical interaction of Fhl2 and Runx2.
   Bacterially expressed fusion protein of Fhl2 and GST and *in vitro* translated and labeled Runx2 can be detected in a pulldown. Runx2 does not unspecifically interact with GST alone.

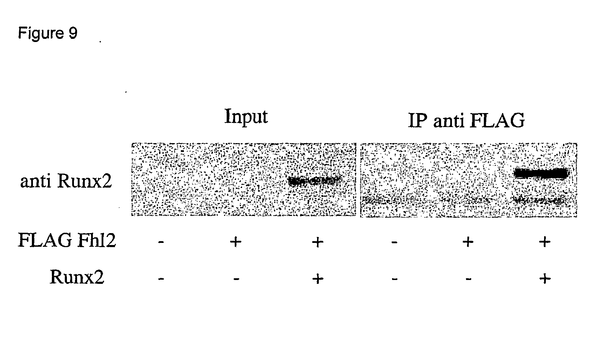

Figure 9 *In vivo* interaction of Fhl2 with Runx2
   Co-Immunoprecipitation of FLAG-tagged Fhl2 and Runx2.

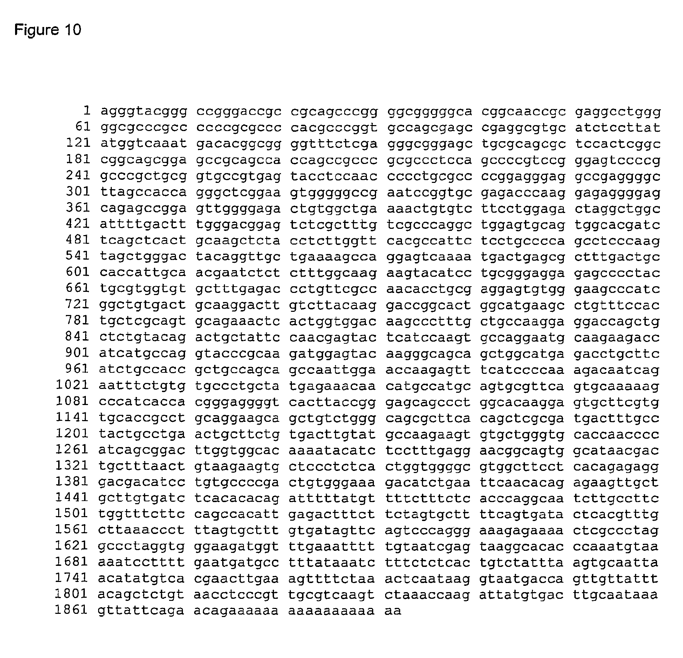

Figure 10 shows the nucleotide sequence of the human Fhl2 gene (Accession No. NM_001450; SEQ ID NO:3). The sequence includes the coding sequence as well as 5'- and 3'-noncoding sequences.

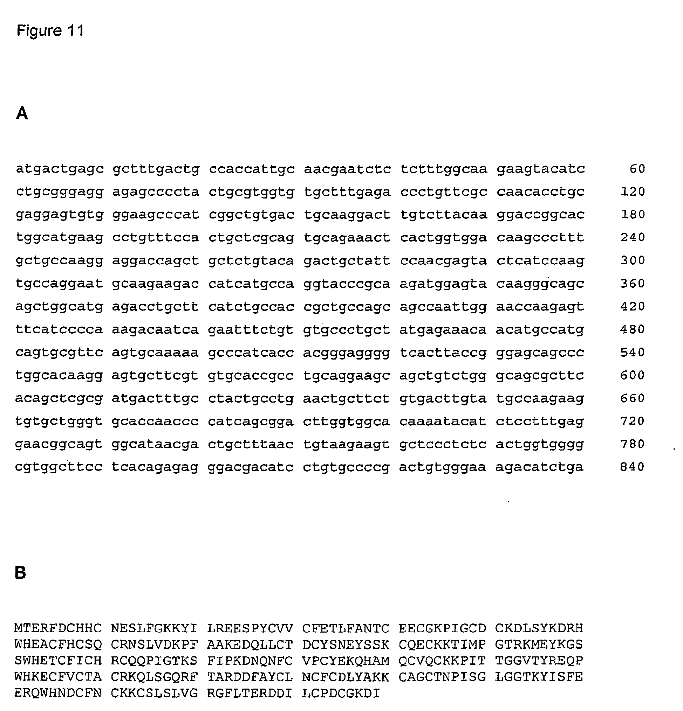

Figure 11 shows the coding sequence of the human Fhl2 gene (A; SEQ ID NO:1) and the amino acid sequence encoded by the human Fhl2 gene (B; SEQ ID NO:2).

The following examples further illustrate the invention.

### Example 1: Analysis of Fhl^{-/-} mice

Fhl2-deficient mice can be generated as described in Kong et al. 2001, Circulation 103: 2731-2738.

### Histology and histomorphometry:

For LacZ stain bone was fixed in 0.2% formaldehyde in 0.1M PIPES buffer pH 6.9, 2mM MgCl₂, 5mM EGTA overnight, incubated in 30% sucrose containing 2mM MgCl₂ and embedded in OCT on dry ice. Cryosections were stained overnight with X-Gal, washed and mounted.

Histological analysis was performed on undecalcified sections stained with von Kossa reagent and counterstained with Kernechtrot (Amling et al., 1999). In vivo double labeling has been performed as described (Amling et al., 1999). Two calcein injections (25mg/kg body weight) were applied in an interval of seven days. The mice were sacrificed two days later. The bones were dissected and fixed in 4% PBS-buffered formaldehyde. After embedding in methylmethacrylate and sectioning at 6µm, a blind analysis of the unstained samples was conducted under fluorescent light. Histomorphometric analyses were performed according to standard protocols (Parfitt et al., 1987) using the Osteomeasure Analysis System (Osteometrics, Atlanta).

### Results:

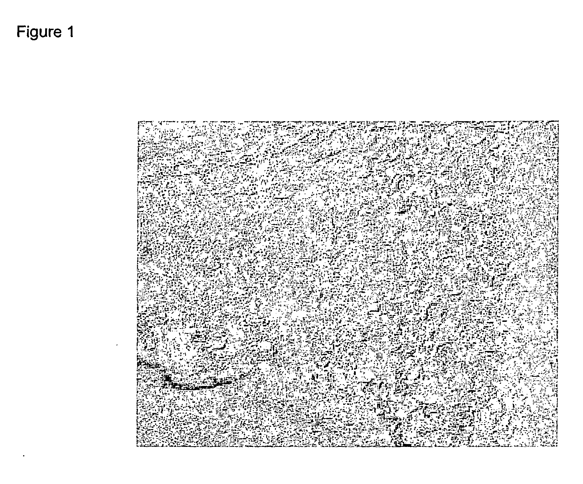

Analysis of the Fhl2 distribution in the mouse revealed that Fhl2 is also expressed in the adult bone (Figure 1). No alteration of the structure or size of the skeleton could be detected, which would have been an indication of aberrant pattern formation and/or modified growth.

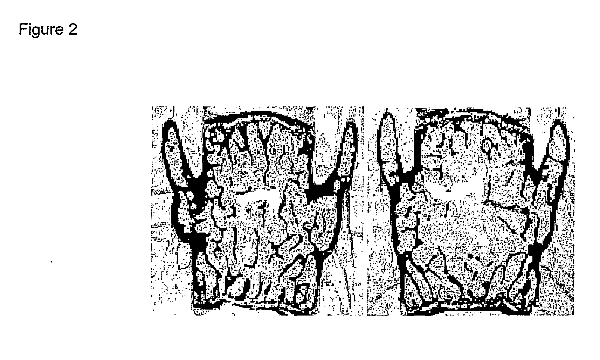

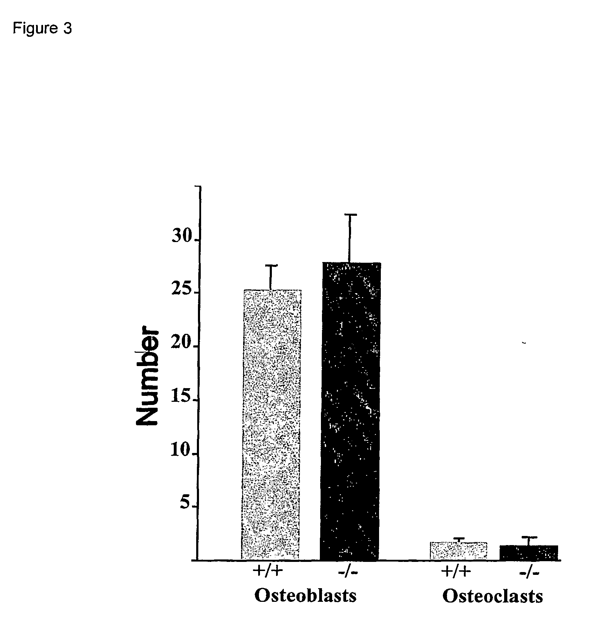

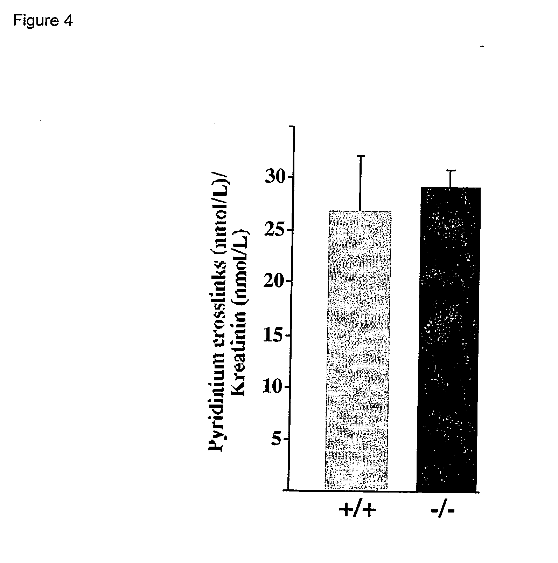

The comparison of vertebral bodies, however, revealed a dramatic decrease of the amount of the calcified extracellular matrix by 30% in male and female Fhl2-deficient animals (Figure 2). The number of osteoblasts as well as the number of osteoclasts in 'knockout'-mice is not altered in comparison to wild-type mice (Figure 3). Analysis of the osteoclast activity also exhibited no significant abnormality (Figure 4). An *in vivo* double labeling using a fluorescent dye in an interval of seven days revealed an articulate decreased amount of newly build extracellular matrix (Figure 5). This difference in the bone formation rate derived from the significantly diminished activity of Fhl2-deficient osteoblasts accounted for 19%.

### Example 2: Cell culture experiments

### Materials and Methods

### Plasmids and reagents

The following plasmids were described previously: pCMX FhIL2, pGEX Fhl2 (Müller *et al.,* 2000); pCMV5 Cbfa1 (Meyers et al., 1995); p6OSE2-luc (Ducy and Karsenty, 1995); hOC-luc (Yeung et al., 2002). The pGEM Cbfa1 was a kind gift of Florian Otto (Freiburg Medical Center). Osteoclast activity was determined using the Metra PYD EIA and Metra Creatinine Assay kits (Quidel).

### Cell lines and transfections

Cos-7 cells were cultured in DMEM supplemented with 10% fetal calf serum. Transient transfection assays were carried out in 12-well plates (1x10⁵ cells per well). Cells were transfected using the calcium phosphate precipitation method as described (Müller *et al.,* 2000). Total amount of transfected DNA was kept constant (4µg) by adding the corresponding amounts of empty expression plasmids and pUC18. 500ng of reporter plasmids were transfected per well. Luciferase activity was assayed 24-30 h after transfection as described (Müller *et al.,* 2000).

### GST pulldown assays

Expression of the GST fusion protein (Pharmacia) and the in vitro transcription-translation reactions (Promega) were performed according to the manufacturer's instructions. GST pulldown assays were performed as described (Dörflinger et al., 1999) using buffer containing 150 mM KCI at 37°C. 10% of the in vitro translated protein was loaded as input.

### Coimmunoprecipitation assays and Western blot analyses

293 cells were transfected with 5µg of pCMV5 Cbfa1 and 5µg pCMX-Flag-Flirt1. Following preclearing with a 40µl 1:1 slurry of GammaBind-Sepharose (Pharmacia), supernatants were incubated for 2.5h with M5 α-Flag antibody (Sigma) and protein extract of the transfected cells. Beads were washed 5 times with WB (10mM Tris-HCl, pH 8.0, 250mM NaCl, 0.5% NP-40, 0,1µg/µl bovine serum albumin, 0.5mM Pefabloc) and analyzed on a 10% SDS gel. Western blots were decorated with an α-PEBP2αA1 (Santa Cruz) antibody. Secondary antibody and chemoluminescence procedure was performed according to the manufacturer (Amersham).

### Results

Runt related protein 2 (Runx2, also referred to as Cbfa1, Osf2, AML3 or PEBP2αA1) is essential for the differentiation of precursors to become mature osteoblasts. Hence, *Runx2-*deficient mice lack bone completely whereas haplo-insufficiency caused cleidocranial dysplasia in men and mice. Moreover, it could be demonstrated in transgenic mice that Runx2 increases osteoblast activity without affecting osteoclast number or activity (Ducy et al. 1999).

In transient transfections in cell culture Fhl2 can increase the transcriptional activity of Runx2 by the factor of three. This super-activation of Runx2 by Fhl2 could not only be demonstrated using an artificial promoter comprising six copies of binding sites for Runx2 in front of a luciferase reporter gene (Figure 6) but also using the promoter of the human osteocalcin gene, a natural target for Runx2 (Figure 7).

The superactivation of Runx2 by Fhl2 could be accomplished either by direct interaction of both proteins or indirectly. In a pulldown it could be shown that Fhl2 physically interacts with Runx2 *in vitro* (Figure 8).

The interaction of Fhl2 and Runx2 can also be shown *in vivo* in immunoprecipitations by transfection of 293 cells with FLAG-tagged Fhl2 and Runx2.

### Literature

• Amling M., Priemel M., Holzmann T., Chapin K., Rueger J.M., Baron R., and Demay M.B. (1999). Rescue of the skeletal phenotype of vitamin D receptor-ablated mice in the setting of normal mineral ion homeostasis: histomorphometric and biomechanical analyses. Endocrinology. 140:4982-4987.
• Dörflinger U., Pscherer A., Moser M., Rummele P., Schüle R., and Buettner R. (1999). Activation of somatostatin receptor II expression by transcription factors MIBP1 and SEF-2 in the murine brain. Mol. Cell. Biol. 19:3736-3747.
• Ducy P., and Karsenty G. (1995). Two distinct osteoblast-specific cis-acting elements control expression of a mouse osteocalcin gene. Mol. Cell. Biol. 15:1858-1869.
• Ducy P., Starbuck M., Priemel M., Shen J., Pinero G., Geoffroy V., Amling M., and Karsenty G. (1999). A Cbfa1-dependent genetic pathway controls bone formation beyond embryonic development. Genes Dev. 13:1025-1036.
• Ducy P. (2000). Cbfa1: a molecular switch in osteoblast biology. Dev. Dyn. 19:461-471.
• Karsenty G., and Wagner E.F. (2002). Reaching a genetic and molecular understanding of skeletal development. Dev. Cell. 2:389-406.
• Meyers S., Lenny N., and Hiebert S.W. (1995). The t(8;21) fusion protein interferes with AML-1B-dependent transcriptional activation. Mol. Cell. Biol. 15:1974-1982.
• Müller J.M., lsele U., Metzger E., Rempel A., Moser M., Pscherer A., Breyer T., Holubarsch C., Buettner R., and Schüle R. (2000). FHL2, a novel tissue-specific coactivator of the androgen receptor. EMBO J. 19:359-369.
• Müller J.M., Metzger E., Greschik H., Bosserhoff A.K., Mercep L., Buettner R., and Schüle R. (2002). The transcriptional coactivator FHL2 transmits Rho signals from the cell membrane into the nucleus. EMBO J. 21:736-748.
• Parfitt A.M., Drezner M.K., Glorieux F.H., Kanis J.A., Malluche H., Meunier P.J., Ott S.M., and Recker R.R. (1987). Bone histomorphometry: standardization of nomenclature, symbols, and units. Report of the ASBMR Histomorphometry Nomenclature Committee. J. Bone Miner. Res. 2:595-610.
• Teitelbaum S.L. (2000). Bone resorption by osteoclasts. Science. 289:1504-1508.
• Yeung F., Law W.K., Yeh C.H., Westendorf J.J., Zhang Y., Wang R., Kao C., and Chung LW. (2002) Regulation of human osteocalcin promoter in hormone-independent human prostate cancer cells. J. Biol. Chem. 277:2468-2476.

## Claims

1. A method for identifying a compound that promotes the activity of osteoblasts, comprising:
(a) contacting a test compound with at least one cell *in vitro*;
(b) determining the level of interaction between Fhl2 protein and Runx2 protein in the cell(s);
(c) comparing the level of interaction determined in (b) to the level of interaction between Fhl2 protein and Runx2 protein in at least one control cell that has not been contacted with the test compound; and
(d) selecting the compound if the level of interaction measured in (b) is significantly different from that in the control cell(s).

2. A method according to claim 1, wherein the at least one cell is selected from the group consisting of primary osteoblasts, MC3T3-E1 cells, ROS17 cells and U2-OS cells.

3. The use of an *Fhl2* nucleic acid for the manufacture of a medicament for the treatment of a bone disease wherein the *Fhl2* nucleic acid is selected from the group consisting of
(a) polynucleotides comprising the sequence as shown in SEQ ID NO:1;
(b) polynucleotides comprising a sequence which has an identity of at least 50% to the sequence as shown in SEQ ID NO:1;
(c) polynucleotides hybridizing to the sequence as shown in SEQ ID NO:1 under stringent conditions;
(d) polynucleotides comprising a sequence which encodes a polypeptide having an amino acid sequence as shown in SEQ ID NO:2; and
(e) polynucleotides comprising a sequence which encodes a polypeptide having an amino acid sequence which has an identity of at least 70% to the amino acid sequence as shown in SEQ ID NO:2.

4. The use according to claim 3, wherein the *Fhl2* nucleic acid is a polynucleotide encoding a polypeptide having an amino acid sequence as shown in SEQ ID NO:2.

5. The use according to claim 3 or 4, wherein the *Fhl2* nucleic acid is a polynucleotide comprising the sequence as shown in SEQ ID NO:1.

6. The use according to any one of claims 3 to 5, wherein the bone disease is **characterized by** a decreased bone mass relative to that of non-diseased bone.

7. The use according to any one of claims 3 to 6, wherein the bone disease is osteoporosis.

8. The use according to any one of claims 3 to 7, wherein the *Fhl2* nucleic acid is overexpressed in osteoblasts.

9. A method of diagnosing a bone disease, comprising
(a) determining *in vitro* the level of expression of the *Fh*/*2* gene in tissue from an individual; and
(b) comparing the level determined in (a) to the level of expression of the *Fhl2* gene in control tissue;
so that if the level determined in (a) is lower than that of the control, the individual is diagnosed as exhibiting the bone disease.

10. A method according to claim 9, wherein the bone disease is osteoporosis.

11. The use of a transgenic non-human animal **characterized by** a decreased level of expression of the Fhl2 gene relative to that of the corresponding wild-type animal as an osteoporosis model.

12. The use according to claim 11 wherein the transgenic non-human animal is a knockout mouse.

## Patentansprüche

1. Verfahren zur Identifizierung einer Verbindung, die die Aktivität von Osteoblasten fördert, umfassend:
(a) das Kontaktieren einer Testverbindung mit mindestens einer Zelle *in vitro;*
(b) das Bestimmen des Interaktionsgrades zwischen Fhl2-Protein und Runx2-Protein in der/den Zelle(n);
(c) das Vergleichen des in (b) bestimmten Interaktionsgrades mit dem Interaktionsgrad zwischen Fhl2-Protein und Runx2-Protein in mindestens einer Kontrollzelle, die nicht mit der Testverbindung kontaktiert worden ist; und
(d) das Auswählen der Verbindung, wenn sich der in (b) gemessene Interaktionsgrad signifikant von dem in der/den Kontrollzelle(n) unterscheidet.

2. Verfahren nach Anspruch 1, wobei die mindestens eine Zelle aus der Gruppe ausgewählt ist, bestehend aus primären Osteoblasten, MC3T3-E1-Zellen, ROS17-Zellen und U2-OS-Zellen.

3. Verwendung einer *Fhl2*-Nukleinsäure zur Herstellung eines Medikaments zur Behandlung einer Knochenerkrankung, wobei die *Fhl2*-Nukleinsäure aus der Gruppe ausgewählt ist, bestehend aus
(a) Polynukleotiden, umfassend die in SEQ ID NR.: 1 gezeigte Sequenz;
(b) Polynukleotiden, umfassend eine Sequenz, die eine Identität von mindestens 50 % mit der in SEQ ID NR.: 1 gezeigten Sequenz aufweist;
(c) Polynukleotiden, die unter stringenten Bedingungen mit der in SEQ ID NR.: 1 gezeigten Sequenz hybridisieren;
(d) Polynukleotiden, umfassend eine Sequenz, die ein Polypeptid mit einer in SEQ ID NR.: 2 gezeigten Aminosäuresequenz kodiert; und
(e) Polynukleotiden, umfassend eine Sequenz, die ein Polypeptid mit einer Aminosäuresequenz, die eine Identität von mindestens 70 % mit der in SEQ ID NR.: 2 gezeigten Aminosäuresequenz hat, kodiert.

4. Verwendung nach Anspruch 3, wobei die *Fhl2*-Nukleinsäure ein Polynukleotid ist, das ein Polypeptid mit einer in SEQ ID NR.: 2 gezeigten Aminosäuresequenz kodiert.

5. Verwendung nach Anspruch 3 oder 4, wobei die *Fhl2*-Nukleinsäure ein Polynukleotid ist, das die in SEQ ID NR.: 1 gezeigte Sequenz umfaßt.

6. Verwendung nach einem der Ansprüche 3 bis 5, wobei die Knochenerkrankung durch eine verringerte Knochmasse, bezogen auf die eines nicht erkrankten Knochens, **gekennzeichnet** ist.

7. Verwendung nach einem der Ansprüche 3 bis 6, wobei die Knochenerkrankung Osteoporose ist.

8. Verwendung nach einem der Ansprüche 3 bis 7, wobei die *Fhl2*-Nukleinsäure in Osteoblasten überexprimiert wird.

9. Verfahren zur Diagnose einer Knochenerkrankung, umfassend
(a) das Bestimmen des Expressionsniveaus des *Fhl2*-Gens in Gewebe eines Individuums *in vitro*; und
(b) das Vergleichen des in (a) bestimmten Niveaus mit dem Expressionsniveau des *Fhl2-*Gens in Kontrollgewebe;
so daß, wenn das in (a) bestimmte Niveau geringer ist als das der Kontrolle, das Individuum als die Knochenerkrankung zeigend diagnostiziert wird.

10. Verfahren nach Anspruch 9, wobei die Knochenerkrankung Osteoporose ist.

11. Verwendung eines transgenen, nicht-menschlichen Lebewesens, **gekennzeichnet durch** ein verringertes Expressionsniveau des *Fhl2*-Gens, bezogen auf das des entsprechenden Wildtyp-Lebewesens, als ein Osteoporosemodell.

12. Verwendung nach Anspruch 11, wobei das transgene, nicht-menschliche Lebewesen eine Knockout-Maus ist.

## Revendications

1. Procédé d'identification d'un composé qui favorise l'activité des ostéoblastes, comprenant :
(a) la mise en contact d'un composé d'essai avec au moins une cellule *in vitro* ;
(b) la détermination du niveau d'interaction entre la protéine Fhl2 et la protéine Runx2 dans la ou les cellules ;
(c) la comparaison du niveau d'interaction déterminé au point (b) au niveau d'interaction entre la protéine Fhl2 et la protéine Runx2 dans au moins une cellule témoin qui n'a pas été mise en contact avec le composé d'essai ; et
(d) la sélection du composé si le niveau d'interaction mesuré au point (b) est très différent de celui mesuré pour la ou les cellules témoins.

2. Procédé selon la revendication 1, dans lequel la au moins une cellule est sélectionnée dans le groupe constitué des ostéoblastes primaires, des cellules de la lignée MC3T3-E1, des cellules de la lignée ROS17 et des cellules de la lignée U2-OS.

3. Utilisation d'un acide nucléique *Fhl2* pour la fabrication d'un médicament en vue de traiter une maladie osseuse, dans laquelle l'acide nucléique *Fhl2* est choisie dans le groupe comprenant :
(a) des polynucléotides comprenant la séquence représentée par la SEQ ID N° 1 ;
(b) des polynucléotides comprenant une séquence présentant au moins 50 % d'identité avec la séquence représentée par la SEQ ID N° 1 ;
(c) des polynucléotides qui s'hybrident avec la séquence représentée par la SEQ ID N° 1 dans des conditions stringentes ;
(d) des polynucléotides comprenant une séquence qui code pour un polypeptide ayant une séquence d'acides aminés représentée par la SEQ ID N° 2 ; et
(e) des polynucléotides comprenant une séquence qui code pour un polypeptide ayant une séquence d'acides aminés qui présente au moins 70 % d'identité avec la séquence d'acides aminés représentée par la SEQ ID N° 2.

4. Utilisation selon la revendication 3, dans laquelle l'acide nucléique *Fhl2* est un polynucléotide codant pour un polypeptide présentant une séquence d'acide aminé représentée par la SEQ ID N °2.

5. Utilisation selon la revendication 3 ou 4, dans laquelle l'acide nucléique *Fhl2* est un polynucléotide comprenant la séquence représentée par la SEQ ID N° 1.

6. Utilisation selon l'une quelconque des revendications 3 à 5, dans laquelle la maladie osseuse est **caractérisée par** une diminution de la masse osseuse en comparaison de celle d'un os non affecté par la maladie.

7. Utilisation selon l'une quelconque des revendications 3 à 6, dans laquelle la maladie osseuse est l'ostéoporose.

8. Utilisation selon l'une quelconque des revendications 3 à 7, dans laquelle l'acide nucléique *Fhl2* est surexprimé dans des ostéoblastes.

9. Procédé pour diagnostiquer une maladie osseuse, comprenant les étapes consistant à :
(a) déterminer *in vitro* le niveau d'expression du gène *Fhl2* dans un tissu prélevé sur un individu ; et
(b) comparer le niveau déterminé au point (a) au niveau d'expression du gène *Fhl2* dans un tissu témoin ;
de sorte que, si le niveau déterminé au point (a) est inférieur à celui déterminé pour le témoin, on diagnostique chez le patient la présence d'une maladie osseuse.

10. Procédé selon la revendication 9, dans lequel la maladie osseuse est l'ostéoporose.

11. Utilisation d'un animal transgénique différent de l'être humain, **caractérisé par** un niveau d'expression du gène *Fhl2* diminué en comparaison de celui de l'animal de type sauvage correspondant, comme modèle de l'ostéoporose.

12. Utilisation selon la revendication 11, dans laquelle l'animal transgénique non de type humain, est une souris dite inactivée (knockout).


### SEQUENCE LISTING

<110> Universitätsklinikum Freiburg
<120> Modulation of Osteoblast Activity by Fhl2
<130> Fhl2
<160> 3
<170> PatentIn version 3.1
<210> 1
   <211> 840
   <212> DNA
   <213> homo sapiens
<400> 1
   atgactgagc gctttgactg ccaccattgc aacgaatctc tctttggcaa gaagtacatc      60
   ctgcgggagg agagccccta ctgcgtggtg tgctttgaga ccctgttcgc caacacctgc     120
   gaggagtgtg ggaagcccat cggctgtgac tgcaaggact tgtcttacaa ggaccggcac     180
   tggcatgaag cctgtttcca ctgctcgcag tgcagaaact cactggtgga caagcccttt     240
   gctgccaagg aggaccagct gctctgtaca gactgctatt ccaacgagta ctcatccaag     300
   tgccaggaat gcaagaagac catcatgcca ggtacccgca agatggagta caagggcagc     360
   agctggcatg agacctgctt catctgccac cgctgccagc agccaattgg aaccaagagt     420
   ttcatcccca aagacaatca gaatttctgt gtgccctgct atgagaaaca acatgccatg     480
   cagtgcgttc agtgcaaaaa gcccatcacc acgggagggg tcacttaccg ggagcagccc     540
   tggcacaagg agtgcttcgt gtgcaccgcc tgcaggaagc agctgtctgg gcagcgcttc     600
   acagctcgcg atgactttgc ctactgcctg aactgcttct gtgacttgta tgccaagaag     660
   tgtgctgggt gcaccaaccc catcagcgga cttggtggca caaaatacat ctcctttgag     720
   gaacggcagt ggcataacga ctgctttaac tgtaagaagt gctccctctc actggtgggg     780
   cgtggcttcc tcacagagag ggacgacatc ctgtgccccg actgtgggaa agacatctga     840
<210> 2
   <211> 279
   <212> PRT
   <213> homo sapiens
<400> 2
   Met Thr Glu Arg Phe Asp Cys His His Cys Asn Glu Ser Leu Phe Gly
   1               5                   10                  15
   Lys Lys Tyr Ile Leu Arg Glu Glu Ser Pro Tyr Cys Val Val Cys Phe
               20                  25                  30
   Glu Thr Leu Phe Ala Asn Thr Cys Glu Glu Cys Gly Lys Pro Ile Gly
           35                  40                  45
   Cys Asp Cys Lys Asp Leu Ser Tyr Lys Asp Arg His Trp His Glu Ala
       50                  55                  60
   Cys Phe His Cys Ser Gln Cys Arg Asn Ser Leu Val Asp Lys Pro Phe
   65                  70                  75                  80
   Ala Ala Lys Glu Asp Gln Leu Leu Cys Thr Asp Cys Tyr Ser Asn Glu
                   85                  90                  95
   Tyr Ser Ser Lys Cys Gln Glu Cys Lys Lys Thr Ile Met Pro Gly Thr
               100                 105                 110
   Arg Lys Met Glu Tyr Lys Gly Ser Ser Trp His Glu Thr Cys Phe Ile
           115                 120                 125
   Cys His Arg Cys Gln Gln Pro Ile Gly Thr Lys Ser Phe Ile Pro Lys
       130                 135                 140
   Asp Asn Gln Asn Phe Cys Val Pro Cys Tyr Glu Lys Gln His Ala Met
   145                 150                 155                 160
   Gln Cys Val Gln Cys Lys Lys Pro Ile Thr Thr Gly Gly Val Thr Tyr
                   165                 170                 175
   Arg Glu Gln Pro Trp His Lys Glu Cys Phe Val Cys Thr Ala Cys Arg
               180                 185                 190
   Lys Gln Leu Ser Gly Gln Arg Phe Thr Ala Arg Asp Asp Phe Ala Tyr
           195                 200                 205
   Cys Leu Asn Cys Phe Cys Asp Leu Tyr Ala Lys Lys Cys Ala Gly Cys
       210                 215                 220
   Thr Asn Pro Ile Ser Gly Leu Gly Gly Thr Lys Tyr Ile Ser Phe Glu
   225                 230                 235                 240
   Glu Arg Gln Trp His Asn Asp Cys Phe Asn Cys Lys Lys Cys Ser Leu
                   245                 250                 255
   Ser Leu Val Gly Arg Gly Phe Leu Thr Glu Arg Asp Asp Ile Leu Cys
               260                 265                 270
   Pro Asp Cys Gly Lys Asp Ile
           275
<210> 3
   <211> 1892
   <212> DNA
   <213> homo sapiens
<400> 3
   agggtacggg ccgggaccgc cgcagcccgg ggcgggggca cggcaaccgc gaggcctggg     60
   ggcgcccgcc ccccgcgccc cacgcccggt gccagcgagc cgaggcgtgc atctccttat    120
   atggtcaaat gacacggcgg ggtttctcga gggcgggagc tgcgcagcgc tccactcggc    180
   cggcagcgga gccgcagcca ccagccgccc gcgccctcca gccccgtccg ggagtccccg    240
   gcccgctgcg gtgccgtgag tacctccaac cccctgcgcc ccggagggag gccgaggggc    300
   ttagccacca gggctcggaa gtgggggccg aatccggtgc gagacccaag gagaggggag    360
   cagagccgga gttggggaga ctgtggctga aaactgtgtc ttcctggaga ctaggctggc    420
   attttgactt tgggacggag tctcgctttg tcgcccaggc tggagtgcag tggcacgatc    480
   tcagctcact gcaagctcta cctcttggtt cacgccattc tcctgcccca gcctcccaag    540
   tagctgggac tacaggttgc tgaaaagcca ggagtcaaaa tgactgagcg ctttgactgc    600
   caccattgca acgaatctct ctttggcaag aagtacatcc tgcgggagga gagcccctac    660
   tgcgtggtgt gctttgagac cctgttcgcc aacacctgcg aggagtgtgg gaagcccatc    720
   ggctgtgact gcaaggactt gtcttacaag gaccggcact ggcatgaagc ctgtttccac    780
   tgctcgcagt gcagaaactc actggtggac aagccctttg ctgccaagga ggaccagctg    840
   ctctgtacag actgctattc caacgagtac tcatccaagt gccaggaatg caagaagacc    900
   atcatgccag gtacccgcaa gatggagtac aagggcagca gctggcatga gacctgcttc    960
   atctgccacc gctgccagca gccaattgga accaagagtt tcatccccaa agacaatcag   1020
   aatttctgtg tgccctgcta tgagaaacaa catgccatgc agtgcgttca gtgcaaaaag   1080
   cccatcacca cgggaggggt cacttaccgg gagcagccct ggcacaagga gtgcttcgtg   1140
   tgcaccgcct gcaggaagca gctgtctggg cagcgcttca cagctcgcga tgactttgcc   1200
   tactgcctga actgcttctg tgacttgtat gccaagaagt gtgctgggtg caccaacccc   1260
   atcagcggac ttggtggcac aaaatacatc tcctttgagg aacggcagtg gcataacgac   1320
   tgctttaact gtaagaagtg ctccctctca ctggtggggc gtggcttcct cacagagagg   1380
   gacgacatcc tgtgccccga ctgtgggaaa gacatctgaa ttcaacacag agaagttgct   1440
   gcttgtgatc tcacacacag atttttatgt tttctttctc acccaggcaa tcttgccttc   1500
   tggtttcttc cagccacatt gagactttct tctagtgctt ttcagtgata ctcacgtttg   1560
   cttaaaccct ttagtgcttt gtgatagttc agtcccaggg aaagagaaaa ctcgccctag   1620
   gccctaggtg ggaagatggt ttgaaatttt tgtaatcgag taaggcacac ccaaatgtaa   1680
   aaatcctttt gaatgatgcc tttataaatc tttctctcac tgtctattta agtgcaatta   1740
   acatatgtca cgaacttgaa agttttctaa actcaataag gtaatgacca gttgttattt   1800
   acagctctgt aacctcccgt tgcgtcaagt ctaaaccaag attatgtgac ttgcaataaa   1860
   gttattcaga acagaaaaaa aaaaaaaaaa aa                                 1892